# EUROPEAN PATENT SPECIFICATION

(11) **EP 2 089 433 B1**
(45) Date of publication and mention of the grant of the patent: **18.05.2011**
(21) Application number: 07824846.5
(22) Date of filing: 12.10.2007
(51) Int. Cl.: C07K 16/40, C07K 16/28, A61K 39/395, A61P 35/00, G01N 33/574, C12N 9/64

(54) **Therapy targeting cathepsin s**
Gegen Cathepsin S gerichtete Therapie
Thérapie dirigée vers Cathepsin S

(30) Priority: 12.10.2006 GB 0620255; 10.04.2007 WO PCT/GB2007/001312
(43) Date of publication of application: 19.08.2009
(73) Proprietor: Fusion Antibodies Limited, Dunmurry, Belfast BT17 0QL (GB)
(72) Inventor: OLWILL, Shane, Belfast BT17 0QL (GB); SCOTT, Christopher, Belfast BT17 0QL (GB); JOHNSTON, James, Belfast BT17 0QL (GB); BURDEN, Roberta, Belfast BT17 0QL (GB)
(74) Representative: Main, Malcolm Charles
(86) International application number: PCT/GB2007/050634
(87) International publication number: WO 2008/044076

(56) References cited:
- WO-A-2007/128987
- WO-A2-2006/109045
- US-A- 5 736 357
- US-A1- 2003 143 714
- KOS J ET AL: "Cathepsin S in tumours, regional lymph nodes and sera of patients with lung cancer: relation to prognosis" BRITISH JOURNAL OF CANCER, LONDON, GB, vol. 85, no. 8, 19 October 2001 (2001-10-19), pages 1193-1200, XP002455887 ISSN: 0007-0920
- JOYCE JOHANNA A ET AL: "Cathepsin cysteine proteases are effectors of invasive growth and angiogenesis during multistage tumorigenesis" CANCER CELL, XX, US, vol. 5, no. 5, 1 May 2004 (2004-05-01), pages 443-453, XP009086749 ISSN: 1535-6108
- FALGUEYRET J-P ET AL: "An activity-based probe for the determination of cysteine cathepsin protease activities in whole cells" ANALYTICAL BIOCHEMISTRY, ACADEMIC PRESS INC. NEW YORK, vol. 335, no. 2, 15 December 2004 (2004-12-15), pages 218-227, XP004642079 ISSN: 0003-2697

## Description

### Field of the Invention

This application relates to methods of treatment of conditions and diseases related to angiogenesis, and compositions for use in such methods.

### Background to the Invention

Angiogenesis, the development of microvasculature, is an integral process within many normal physiological processes such as normal development and wound healing. Angiogenesis is characterised by the stimulation of endothelial cells to form primary blood vessels where a non-clarified complex interplay exists between the endothelial cells, surrounding microenvironment and a range of pro and anti-angiogenic factors. However, uncontrolled or inappropriate angiogenesis is accepted as an underlying factor to the pathology of a wide range of diseases including tumour progression and ocular disease.

### Pathological Ocular Neovascularisation

Unregulated angiogenesis in the eye is considered the leading cause of blindness, in a wide range of conditions including corneal graft rejection, neovascularization following injury or infection, diabetic retinopathy, retrolental fibroplasia and neovascular glaucoma.

Molecules such as vascular endothelial growth factor (VEGF) have been shown to play instrumental roles in promotion of angiogenesis and indeed the sequestering of this molecule using antibodies has been established as a therapeutic strategy for the prevention of pathological angiogenesis.

However, other proteins, such as cathepsin S, have also been shown to be upregulated in angiogenic sites.

Cathepsin S (Cat S) is a member of the papain superfamily of lysosomal cysteine proteases. To date, eleven human cathepsins have been identified, but the specific *in vivo* roles of each are still to be determined (Katunuma *et al*, 2003). Cathepsins B, L, H, F, O, X and C are expressed in most cells, suggesting a possible role in regulating protein turnover, whereas Cathepsins S, K, W and V are restricted to particular cells and tissues, indicating that they may have more specific roles (Kos *et al,* 2001; Berdowska, 2004).

Cat S was originally identified from bovine lymph nodes and spleen and the human form cloned from a human macrophage cDNA library (Shi *et al*, 1992). The gene encoding Cat S is located on human chromosome 1q21. The 996 base pair transcript encoded by the Cat S gene, is initially translated into an unprocessed precursor protein with a molecular weight of 37.5 kDa. The unprocessed protein is composed of 331 amino acids; a 15 amino acid signal peptide, a 99 amino acid pro-peptide sequence and a 217 amino acid peptide. Cat S is initially expressed with a signal peptide that is removed after it enters the lumen of the endoplasmic reticulum. The propeptide sequence binds to the active site of the protease, rendering it inactive until it has been transported to the acidic endosomal compartments, after which the propeptide sequence is removed and the protease is activated (Baker *et al,* 2003).

Cat S has been identified as a key enzyme in major histocompatibility complex class II (MHC-II) mediated antigen presentation, by cleavage of the invariant chain, prior to antigen loading. Studies have shown that mice deficient in Cat S have an impaired ability to present exogenous proteins by APC's (Nakagawa *et al*, 1999). The specificity of Cat S in the processing of the invariant chain Ii, allows for Cat S specific therapeutic agents in the treatment of conditions such as asthma and autoimmune disorders (Chapman *et al*, 1997).

### Pathological association of Cat S

Alterations in protease control frequently underlie many human pathological processes. The deregulated expression and activity of the lysosomal cysteine protease Cathepsin S has been linked to a range of conditions including neurodegenerative disorders, autoimmune diseases and certain malignancies.

Cat S upregulation has been linked to several neurodegenerative disorders. It is believed to have a role in the production of the β peptide (Aβ) from the amyloid precursor protein (APP) (Munger *et al,* 1995) and its expression has been shown to be upregulated in both Alzheimer's Disease and Down's Syndrome (Lemere *et al*, 1995). Cat S may also have a role in Multiple Sclerosis through the ability of Cat S to degrade myelin basic protein, a potential autoantigen implicated in the pathogenesis of MS (Beck *et al,* 2001) and in Creutzfeldt - Jakob disease (CJD) patients, Cat S expression has been shown to increase more than four fold (Baker *et al,* 2002).

Aberrant Cat S expression has also been associated with atherosclerosis. Cat S expression is negligible in normal arteries, yet human atheroma display strong immunoreactivity (Sukhova *et al*, 1998). Further studies using knockout mice, deficient in both Cat S and the LDL-receptor, were shown to develop significantly less atherosclerosis (Sukhova *et al*, 2003). Further research has linked Cat S expression with inflammatory muscle disease and rheumatoid arthritis. Muscle biopsy specimens from patients with inflammatory myopathy had a 10 fold increase in Cat S expression compared to control muscle sections (Wiendl *et al,* 2003), and levels of Cat S expression were significantly higher in synovial fluid from patients with rheumatoid arthritis compared to those with osteoarthritis (Hashimoto *et al*, 2001).

The association of CatS with angiogenesis was first shown *in vitro* using CatS deficient endothelial cells (Shi et al, 2003). Microvascular endothelial cells (ECs) have been shown to secrete proteases, permitting penetration of the vascular basement membrane as well as the interstitial extracellular matrix. Treatment of cultured ECs with inflammatory cytokines or angiogenic factors stimulated expression of CatS, and its inhibition reduced microtubule formation. CatS -/- mice displayed defective microvessel development during wound repair in comparison to wild-type controls (Shi et al, 2003).

Further examination of the role of CatS in angiogenesis and tumour growth was demonstrated in a transgenic mouse model for pancreatic islet cell carcinoma. CatS-/- mice were found to develop significantly smaller tumours and fewer angiogenic islets in comparison to the CatS+/+ control mice (Gocheva *et al*., 2006). Insight to the molecular mechanism underpinning this phenotype was subsequently provided by evidence that CatS could cleave and inactivate anti-angiogenic peptides and promote the generation of active pro-angiogenic fragments (Wang et al, 2006).

Finally, the role of CatS in malignancy and angiogenesis has also been examined in a murine model of hepatocellular carcinoma. Microarray analysis on normal ECs and those extracted from tumour model showed that CatS gene expression was 74-fold greater in the tumour ECs compared to the normal cells (Ryschich et al, 2006). Kos et al. (British Journal of Cancer, 85(8) pp 1193-1200 (2001)) describes immunohistochemical analysis of tumours, regional lymph nodes and sera of patients with lung cancer using monoclonal anti-human CatS antibodies. Joyce et al (Cancer Cell, 5(5) pp 443-453 (2004)) describes the use of gene expression profiling to demonstrate that cathepsin activity is increased during tumorigenesis.

The generation of inhibitors specifically targeting the proteolytic activity of Cat S have potential as therapeutic agents for alleviations of the symptoms associated with the activity of this protease.

### Inhibition of Cat S

When proteases are over-expressed, therapeutic strategies have focused on the development of inhibitors to block the activity of these enzymes. The generation of specific small molecule inhibitors to the cathepsins have proved difficult in the past, due to problems with selectivity and specificity. The dipeptide α-keto-β-aldehydes developed as potent reversible inhibitors to Cat S by Walker *et al*, had the ability to inhibit Cat B and L, albeit with less efficiency (Walker *et al*, 2000), and the Cat S inhibitor 4-Morpholineurea-Leu-HomoPhe-vinylsulphone (LHVS) has also been shown to inhibit other cathepsins when used at higher concentrations (Palmer *et al*, 1995). PCT/GB2006/001314 (W02006/109045), filed 10 April 2006, and which shares an inventor with the present application, describes a monoclonal antibody with specificity for cathepsin S which potently inhibits the proteolytic activity of cathepsin S. This antibody was shown to inhibit tumour cell invasion and angiogenesis. W02006/109045 teaches that this is the first demonstration of a cathepsin S specific antibody directly inhibiting the protease activity of cathepsin S and thus uniquely enables the use of such antibodies as active therapeutic agents with a wide range of applications.

Other than as described in W02006/109045, there have been no reports of anti Cathepsin S antibodies which have demonstrable therapeutic potential. Notably, although some antibodies which bind certain particular proteins have been shown to have some effect on the normal activity of the particular protein, this has not previously been demonstrated for any antibody which binds Cathepsin S. Indeed, in W02006/109045, it is shown that control antibodies which bound cathepsin S but which did not affect proteolytic activity had no effect on tumour cell invasion.

### Summary of the Invention

The present inventors have studied the effects of a number of cathepsin S antibodies on a number of models of disease.

The present inventors have shown for the first time that antibodies, which have specificity for cathepsin S, but which do not inhibit the proteolytic activity of cathepsin S, nevertheless act as potent inhibitors of angiogenesis. This was particularly surprising to the inventors given that, as taught in WO2006/109045, it was believed that to counteract the pathological effects of dysregulation of cathepsin S activity, strategies are required which inhibit the proteolytic effects of cathepsin S. Furthermore, although the use of antibodies to sequester proteins has been suggested as a therapeutic strategy for some proteins, e.g. VEGF, such a strategy has not, until now, been considered as feasible for the treatment of diseases associated with cathepsin S activity.

Accordingly, described herein is a method of treating a condition associated with angiogenesis in a patient in need of treatment thereof, said method comprising administration of an antibody molecule or a nucleic acid encoding said antibody molecule to said patient, wherein said antibody molecule specifically binds cathepsin S but does not inhibit the proteolytic activity of cathepsin S.

According to a first aspect of the invention, there is provided the use of an antibody molecule or nucleic acid encoding the antibody molecule wherein said antibody molecule specifically binds cathepsin S but does not inhibit the proteolytic activity of cathepsin S in the preparation of a medicament for the treatment of cancer, an inflammatory condition, an ocular disease, atherosclerosis, a neurodegenerative disorder, an autoimmune disorder, hemangioma, a solid tumor, leukemia, telangiectasia, psoriasis, scleroderma, pyogenic granuloma, myocardial angiogenesis, Crohn's disease, plaque neovascularization, coronary collaterals, cerebral collaterals, arteriovenous malformations, ischemic limb angiogenesis, arthritis, diabetic neovascularization, peptic ulcer, Helicobacter related disease, a fracture, keloids, and vasculogenesis, ulcerative colitis, or bartonellosis. Said antibody molecule is the antibody molecule of the third aspect of the invention.

The invention may be used in the treatment of any disease or condition in which angiogenesis plays a part in the pathology of the disease. Such conditions and diseases include, but are not limited to cancer, inflammatory conditions, for example inflammatory muscle disease, rheumatoid arthritis and asthma, ocular diseases, atherosclerosis and cancer.

Moreover, the demonstration that antibody molecules which specifically bind cathepsin S but do not inhibit the proteolytic activity of cathepsin S nevertheless inhibit angiogenesis enables the use of such antibody molecules in the treatment of other conditions associated with cathepsin S.

Also described is a method of treating a condition associated with activity of Cathepsin S in a patient in need of treatment thereof, said method comprising administration of an antibody molecule or nucleic acid encoding the antibody molecule to said patient, wherein said antibody molecule specifically binds cathepsin S but does not inhibit the proteolytic activity of cathepsin S.

Also described is the use of an antibody molecule or nucleic acid encoding the antibody molecule wherein said antibody molecule specifically binds cathepsin S but does not inhibit the proteolytic activity of cathepsin S in the preparation of a medicament for the treatment of a condition associated with aberrant activity or expression of cathepsin S.

The invention may be used in the treatment of a condition with which aberrant activity of cathepsin S is associated, in particular conditions associated with expression of cathepsin S. For example, conditions in which the invention may be used include, but are not limited to neurodegenerative disorders, for example Alzheimer's disease and multiple sclerosis, autoimmune disorders, and other diseases associated with excessive, deregulated or inappropriate angiogenesis.

In the invention, any suitable anti-cathepsin S antibody molecule, which does not inhibit the proteolytic activity of cathepsin S, but which inhibits angiogenesis, may be used.

As described in the Examples, the inventors have identified the binding region on Cathepsin S to which the 1E4 antibody specifically binds. The binding region has the amino acid sequence shown as Sequence ID No: 1:
ELPYGREDVLKEAVANKGPVSVGVDARHP (Sequence ID No: 1)

Accordingly, in a second aspect of the invention, there is provided an isolated polypeptide, wherein said polypeptide consists of a polypeptide sequence having at least 90%, homology to Sequence ID No: 1. In one embodiment, the polypeptide sequence consists of a polypeptide sequence having the amino acid sequence shown as Sequence ID No: 1.

Thus, in a third aspect of the invention provides an antibody molecule which specifically binds cathepsin S and which inhibits angiogenesis but does not inhibit the proteolytic activity of cathepsin S, wherein said antibody molecule has binding specificity for the polypeptide of the second aspect of the invention.

In one embodiment of the invention, the antibody molecule is a 1E4 antibody or a fragment or variant thereof.

The antibody molecule may be an antibody, for example a whole antibody. In one alternative embodiment, the antibody molecule may be an antibody fragment such as an scFv.

Related antibody molecules which also inhibit angiogenesis but which do not inhibit the proteolytic activity of cathepsin S and which optionally have similar or greater binding specificity may also be used as the antibody molecule in the present invention.

Such antibody molecules may comprise at least one of the CDRs of the V_{H} chain of the 1E4 antibody and/or at least one of the CDRs of the V_{H} chain of the 1E4 antibody in which 5 or less, for example 4, 3, 2, or 1 amino acid substitutions have been made in at least one CDR and wherein the antibody molecule retains the ability to inhibit angiogenesis but does not have ability to inhibit the proteolytic activity of cathepsin S.

In one embodiment of the invention, the antibody molecule of and for use in the invention has the ability to inhibit tumour cell invasion.

According to a further aspect of the invention, there is provided an antibody molecule according to the third aspect of the invention or a nucleic acid encoding said antibody molecule for use in therapy.

Another aspect of the invention is a pharmaceutical composition comprising an antibody molecule according to the third aspect of the invention or nucleic acid encoding the antibody molecule wherein said antibody molecule specifically binds cathepsin S but does not inhibit the proteolytic activity of cathepsin S.

Preferred and alternative features of each aspect of the invention are as for each of the other aspects mutatis mutandis unless the context demands otherwise.

### Detailed Description

### Antibody molecules

In the context of the present invention, an antibody molecule (or specific binding member) is a molecule which has binding specificity for another molecule, in particular for cathepsin S. The antibody molecule may be an antibody or fragment thereof.

An antibody should be understood to refer to an immunoglobulin or part thereof or any polypeptide comprising a binding domain which is, or is homologous to, an antibody binding domain. Specific antibody molecules include but are not limited to polyclonal, monoclonal, monospecific, polyspecific antibodies and fragments thereof and chimeric antibodies comprising an immunoglobulin binding domain fused to another polypeptide. Antibody mimetics are also encompassed by antibody molecules.

Intact (whole) antibodies comprise an immunoglobulin molecule consisting of heavy chains and light chains, each of which carries a variable region designated VH and VL, respectively. The variable region consists of three complementarity determining regions (CDRs, also known as hypervariable regions) and four framework regions (FR) or scaffolds. The CDR forms a complementary steric structure with the antigen molecule and determines the specificity of the antibody.

Fragments of antibodies may retain the binding ability of the intact antibody and may be used in place of the intact antibody. Accordingly, for the purposes of the present invention, unless the context demands otherwise, the term "antibody molecules" should be understood to encompass antibody fragments. Examples of antibody fragments include Fab, Fab', F (ab')2, Fd, dAb, and Fv fragments, scFvs, bispecific scFvs, diabodies, linear antibodies (see US patent 5, 641, 870, Example 2 ; Zapata etal., Protein Eng 8 (10) : 1057-1062 [1995]) ; single-chain antibody molecules; and multispecific antibodies formed from antibody fragments.

The Fab fragment consists of an entire L chain ( VL and CL), together with VH and CH1. Fab' fragments differ from Fab fragments by having additional few residues at the carboxy terminus of the CH1 domain including one or more cysteines from the antibody hinge region. The F (ab') 2 fragment comprises two disulfide linked Fab fragments.

Fd fragments consist of the VH and CH1 domains.

Fv fragments consist of the VL and VH domains of a single antibody.

Single-chain Fv fragments are antibody fragments that comprise the VH and VL domains connected by a linker which enables the scFv to form an antigen binding site. (see Pluckthun in The Pharmacology of Monoclonal Antibodies, vol.113, Rosenburg and Moore eds., Springer-Verlag, New York, pp. 269-315 (1994).

Diabodies are small antibody fragments prepared by constructing scFv fragments (see preceding paragraph) with short linkers (about 5-10 residues) between the VH and VL domains such that inter-chain but not intra-chain pairing of the V domains is achieved, resulting in a multivalent fragment, i.e. a fragment having two antigen-binding sites (see, for example, EP 404 097 ; WO 93/11161 ; and Hollinger et al., Proc. Natl. Acad. Sci. USA, 90 : 6444-6448 (1993))

Further encompassed by fragments are individual CDRs.

As described above, the antibody molecules of and for use in the present invention are not limited to the 1E4 antibody, but also extends to other antibodies which maintain the ability to inhibit angiogenesis but which do not inhibit the proteolytic activity of Cat S. Thus, the CDR amino acid sequences of such antibodies in which one or more amino acid residues are modified may also be used as the CDR sequence. The modified amino acid residues in the amino acid sequences of the CDR variant are preferably 30% or less, more preferably 20% or less, most preferably 10% or less, within the entire CDR. Such variants may be provided using the teaching of the present application and techniques known in the art. The CDRs may be carried in a framework structure comprising an antibody heavy or light chain sequence or part thereof. Preferably such CDRs are positioned in a location corresponding to the position of the CDR(s) of naturally occurring VH and VL domains. The positions of such CDRs may be determined as described in Kabat et al, Sequences of Proteins of Immunological Interest, US Dept of Health and Human Services, Public Health Service, Nat'1 Inst. of Health, NIH Publication No. 91-3242, 1991 and online at www.kabatdatabase.com http://immuno.bme.nwu.edu.

Furthermore, modifications may alternatively or additionally be made to the Framework Regions of the variable regions. Such changes in the framework regions may improve stability and reduce immunogenicity of the antibody.

The antibodies of the invention herein include "chimeric" antibodies in which a portion of the heavy and/or light chain is identical with or homologous to corresponding sequences in antibodies derived from a particular species or belonging to a particular antibody class or subclass, while the remainder of the chain (s) is identical with or homologous to corresponding sequences in antibodies derived from another species or belonging to another antibody class or subclass, as well as fragments of such antibodies, so long as they exhibit the desired biological activity (see U. S. Patent No. 4, 816, 567 ; and Morrison et al., Proc. Natl. Acad. Sci. USA, 81 : 6851-6855 (1984)). Chimeric antibodies of interest herein include "primatized"antibodies comprising variable domain antigen-binding sequences derived from a non-human primate(e. g. Old World Monkey, Ape etc), and human constant region sequences.

### Production Of Antibodies

Antibody molecules of and for use in the present invention may be produced in any suitable way, either naturally or synthetically. Such methods may include, for example, traditional hybridoma techniques (Kohler and Milstein (1975) Nature, 256 :495-499), recombinant DNA techniques (see e.g. U. S. Patent No. 4,816, 567), or phage display techniques using antibody libraries (see e.g. Clackson et al. (1991) Nature, 352: 624-628 and Marks et al. (1992) Bio/ Technology, 10: 779-783). Other antibody production techniques are described in Antibodies: A Laboratory Manual, eds. Harlow et al., Cold Spring Harbor Laboratory, 1988.

Traditional hybridoma techniques typically involve the immunisation of a mouse or other animal with an antigen in order to elicit production of lymphocytes capable of binding the antigen. The lymphocytes are isolated and fused with a a myeloma cell line to form hybridoma cells which are then cultured in conditions which inhibit the growth of the parental myeloma cells but allow growth of the antibody producing cells. The hybridoma may be subject to genetic mutation, which may or may not alter the binding specificity of antibodies produced. Synthetic antibodies can be made using techniques known in the art (see, for example, Knappik et al, J. Mol. Biol. (2000) 296, 57-86 and Krebs et al, J. Immunol. Meth. (2001) 2154 67-84.

Modifications may be made in the VH, VL or CDRs of the antibody molecules, or indeed in the FRs using any suitable technique known in the art. For example, variable VH and/or VL domains may be produced by introducing a CDR, e.g. CDR3 into a VH or VL domain lacking such a CDR. Marks et al. (1992) Bio/ Technology, 10: 779-783 describe a shuffling technique in which a repertoire of VH variable domains lacking CDR3 is generated and is then combined with a CDR3 of a particular antibody to produce novel VH regions. Using analogous techniques, novel VH and VL domains comprising CDR derived sequences of the present invention may be produced.

Alternative techniques of producing variant antibodies of the invention may involve random mutagenesis of gene(s) encoding the VH or VL domain using, for example, error prone PCR (see Gram et al, 1992, P.N.A.S. 89 3576-3580. Additionally or alternatively, CDRs may be targeted for mutagenesis e.g. using the molecular evolution approaches described by Barbas et al 1991 PNAS 3809-3813 and Scier 1996 J Mol Biol 263 551-567.

Having produced such variants, antibodies and fragments may be tested for binding to Cat S and for the ability to inhibit the proteolytic activity of cathepsin S.

As described herein, the inventors have demonstrated that antibody molecules according to the invention have an anti-angiogenic effect. This therefore enables the use of the antibody molecules of the invention as active therapeutic agents.
Accordingly in one embodiment of the invention, the antibody molecule is a "naked" antibody molecule. A "naked" antibody molecule is an antibody molecule which is not conjugated with an "active therapeutic agent".

In the context of the present application, an "active therapeutic agent" is a molecule or atom which is conjugated to an antibody moiety (including antibody fragments, CDRs etc) to produce a conjugate. Examples of such "active therapeutic agents" include drugs, toxins, radioisotopes, immunomodulators, chelators, boron compounds , dyes, nanoparticles etc.

In another embodiment of the invention, the antibody molecule is in the form of an immunoconjugate, comprising an antibody fragment conjugated to an "active therapeutic agent".

Methods of producing immunoconjugates are well known in the art; for example, see U. S. patent No. 5,057,313, Shih et al., Int. J. Cancer 41: 832-839 (1988); Shih et al., Int. J.Cancer 46: 1101-1106 (1990), Wong, Chemistry Of Protein Conjugation And Cross-Linking (CRC Press 1991); Upeslacis et al., "Modification of Antibodies by Chemical Methods,"in Monoclonal Antibodies: Principles And Applications, Birch et al. (eds.), pages 187-230 (Wiley-Liss, Inc. 1995); Price, "Production and Characterization of Synthetic Peptide-Derived Antibodies," in Monoclonal Antibodies: Production, Engineering And Clinical Application, Ritter et al. (eds.), pages 60-84 (Cambridge University Press 1995).

The antibody molecules of and for use in the invention may comprise further modifications. For example the antibodies can be glycosylated, pegylated, or linked to albumin or a nonproteinaceous polymer. The antibody molecule may be in the form of an immunoconjugate.

Antibodies of the invention may be labelled. Labels which may be used include radiolabels, enzyme labels such as horseradish peroxidase, alkaline phosphatase, or biotin.

As noted above, antibodies of and for use in the methods of the invention do not inhibit the proteolytic effct of cathepsin S. The ability of an antibody molecule to inhibit the proteolytic activity of cathepsin S may be tested using any suitable method. For example the ability of an antibody molecule to inhibit the proteolytic activity of cathepsin S may be tested using a fluorimetric assay. In such an assay, any suitable fluorigenic substrate may be used, for example Cbz-Val-Val-Arg-AMC. An antibody molecule is considered to inhibit the proteolytic activity of cathepsin S if it has the ability to inhibit its activity by a significant amount. For example, in one embodiment, the antibody molecule is considered not to inhibit the proteolytic activity if it inhibits the proteolytic activity by no more than 10%, for example no more than 5%, such as no more than 2%, for example less than 1%, such as 0% compared to an appropriate control antibody known to inhibit the proteolytic effect of cathepsin S.

The ability of an antibody molecule to inhibit angiogenesis may be tested using any suitable assay known in the art. Many in vitro and in vivo assays are known in the art. These include Matrigel plug and corneal neovascularization assays, the in vivo/in vitro chick chorioallantoic membrane (CAM) assay, and the in vitro cellular (proliferation, migration, tube formation) and organotypic (aortic ring) assays, the chick aortic arch and the Matrigel sponge assays. Further details of such assays may be found, for example, in Auerbach et al, Clinical Chemistry 49: 32-40, 2003; 10.1373/49.1.32. Further details are also provided in the Examples.

The ability of an antibody molecule to inhibit tumour cell invasion may be tested using any suitable invasion assay known in the art. For example, such ability may be tested using a modified Boyden chamber as described in the Examples. The antibody molecule may be tested using any suitable tumour cell line, for example a prostate carcinoma cell line, e.g. PC3, an astrocytoma cell line e.g.U251mg, a colorectal carcinoma cell line, e.g. HCT116, or a breast cancer cell line, e.g. MDA-MB-231 or MCF7. An antibody molecule is considered to inhibit tumour cell invasion if it has the ability to inhibit invasion by a statistically significant amount. For example, in one embodiment, the antibody molecule is able to inhibit invasion by at least 10%, for example at least 25%, at least 50%, at least 60%, at least 70%, at least 80% or at least 90% when compared to an appropriate control antibody.

In one embodiment of the invention, an antibody molecule for use in the invention may have inhibitory activity( for example to inhibit the angiogenesis) with a potency of at least 25%, for example at least 40%, for example at least 50% of the inhibitory potency of antibody 1E4.

### Polypeptides

As described above, the inventors have identified the binding region to which the 1E4 antibody binds. Accordingly, the invention extends to an isolated polypeptide, wherein said polypeptide consists of a polypeptide sequence having at least 90% homology, or at least 95% homology to Sequence ID No: 1. In one embodiment, the polypeptide sequence consists of a polypeptide sequence having the amino acid sequence shown as Sequence ID No: 1.
ELPYGREDVLKEAVANKGPVSVGVDARHP (Sequence ID No: 1)

Thus a variant polypeptide in accordance with the present invention may include within the sequence shown as Sequence ID No: 1, a single amino acid change or 2, 3, 4, 5, 6, 7, 8, or 9 changes, or about 10, 15, changes. In addition to one or more changes within the amino acid sequence shown, a variant polypeptide may include additional amino acids at the C terminus and/or N-terminus.

Homology (i.e. similarity or identity) may be as defined using sequence comparisons are made using FASTA and FASTP (see Pearson & Lipman, 1988. Methods in Enzymology 183 : 6398). Parameters are preferably set, using the default matrix, as follows :
Gapopen (penalty for the first residue in a gap) :-12 for proteins/-16 for DNA
Gapext (penalty for additional residues in a gap) :-2 for proteins/-4 for DNA
KTUP word length : 2 for proteins/6 for DNA.
Homology may be at the nucleotide sequence and/or encoded amino acid sequence level.

Naturally, regarding nucleic acid variants, changes to the nucleic acid which make no difference to the encoded polypeptide (i.e.'degeneratively equivalent') are included within the scope of the present invention.

Changes to a sequence, may be by one or more of addition, insertion, deletion or substitution of one or more nucleotides in the nucleic acid, leading to the addition, insertion, deletion or substitution of one or more amino acids in the encoded polypeptide. Changes may be by way of conservative variation, i. e. substitution of one hydrophobic residue such as isoleucine, valine, leucine or methionine for another, or the substitution of one polar residue for another, such as arginine for lysine, glutamic for aspartic acid, or glutamine for asparagine. As is well known to those skilled in the art, altering the primary structure of a polypeptide by a conservative substitution may not significantly alter the activity of that peptide because the side-chain of the amino acid which is inserted into the sequence may be able to form similar bonds and contacts as the side chain of the amino acid which has been substituted out. This is so even when the substitution is in a region which is critical in determining the peptides conformation.

Also included are variants having non-conservative substitutions. As is well known to those skilled in the art, substitutions to regions of a peptide which are not critical in determining its conformation may not greatly affect its activity because they do not greatly alter the peptide's three dimensional structure.

In regions which are critical in determining the peptides conformation or activity such changes may confer advantageous properties on the polypeptide. Indeed, changes such as those described above may confer slightly advantageous properties on the peptide e. g. altered stability or specificity.

### Nucleic Acid

Nucleic acid of and for use in the present invention may comprise DNA or RNA. It may be produced recombinantly, synthetically, or by any means available to those in the art, including cloning using standard techniques.

The nucleic acid may be inserted into any appropriate vector. A vector comprising a nucleic acid of the invention forms a further aspect of the present invention. In one embodiment the vector is an expression vector and the nucleic acid is operably linked to a control sequence which is capable of providing expression of the nucelic acid in a host cell. A variety of vectors may be used. For example, suitable vectors may include viruses (e. g. vaccinia virus, adenovirus,etc.), baculovirus); yeast vectors, phage, chromosomes, artificial chromosomes, plasmids, or cosmid DNA.

The vectors may be used to introduce the nucleic acids of the invention into a host cell. A wide variety of host cells may be used for expression of the nucleic acid of the invention. Suitable host cells for use in the invention may be prokaryotic or eukaryotic. They include bacteria, e.g. E. coli, yeast, insect cells and mammalian cells. Mammalian cell lines which may be used include Chinese hamster ovary cells, baby hamster kidney cells, NSO mouse melanoma cells, monkey and human cell lines and derivatives thereof and many others.
A host cell strain that modulates the expression of, modifies, and/or specifically processes the gene product may be used. Such processing may involve glycosylation, ubiquitination, disulfide bond formation and general post-translational modification.

Accordingly, the present invention also provides a host cell, which comprises one or more nucleic acid or vectors of the invention.

Also encompassed by the invention is a method of production of an antibody molecule of the invention, the method comprising culturing a host cell comprising a nucleic acid of the invention under conditions in which expression of the antibody molecules from the nucleic acid occurs and, optionally, isolating and/or purifying the antibody molecule.

For further details relating to known techniques and protocols for manipulation of nucleic acid, for example, in preparation of nucleic acid constructs, mutagenesis, sequencing, introduction of DNA into cells and gene expression, and analysis of proteins, see, for example, Current Protocols in Molecular Biology, 5th ed.,Ausubel et al. eds., John Wiley & Sons, 2005 and, Molecular Cloning: a Laboratory Manual: 3rd edition Sambrook et al., Cold Spring Harbor Laboratory Press, 2001.

### Treatment

The antibody molecules and nucleic acids of the invention may be used in the treatment of a number of medical conditions.

Treatment" includes any regime that can benefit a human or non-human animal. The treatment may be in respect of an existing condition or may be prophylactic (preventative treatment). Treatment may include curative, alleviation or prophylactic effects.

The antibody molecules and nucleic acids of the invention may be used in the treatment of a variety of condition and disorders. These include atherosclerosis and neoplastic disease, neurodegenerative disorders, autoimmune diseases, cancer, inflammatory disorders, asthma, and atherosclerosis, and pain.

Neurodegenerative disorders which may be treated using the antibody molecules, nucleic acids and methods of the invention include, but are not limited to, Alzheimer's Disease, Multiple Sclerosis and Creutzfeldt - Jakob disease.

Autoimmune diseases for which the invention may be used include inflammatory muscle disease and rheumatoid arthritis.

The antibody molecules, nucleic acids and methods of the invention may also be used in the treatment of cancers.

"Treatment of cancer" includes treatment of conditions caused by cancerous growth and/or vascularisation and includes the treatment of neoplastic growths or tumours. Examples of tumours that can be treated using the invention are, for instance, sarcomas, including osteogenic and soft tissue sarcomas, carcinomas, e.g., breast-, lung-, bladder-, thyroid-, prostate-, colon-, rectum-, pancreas-, stomach-, liver-, uterine-, prostate, cervical and ovarian carcinoma, non-small cell lung cancer, hepatocellular carcinoma, lymphomas, including Hodgkin and non-Hodgkin lymphomas, neuroblastoma, melanoma, myeloma, Wilms tumor, and leukemias, including acute lymphoblastic leukaemia and acute myeloblastic leukaemia, astrocytomas, gliomas and retinoblastomas.

The invention may be particularly useful in the treatment of existing cancer and in the prevention of the recurrence of cancer after initial treatment or surgery.

The antibody molecules, nucleic acids and compositions of the invention may also be used in the treatment of other disorders mediated by or associated with angiogenesis. Such conditions include, for example, tumours, various autoimmune disorders, hereditary disorders, ocular disorders.

Particular ocular disorders associated with angiogenesis which may be treated using the methods and antibody molecules of the invention include corneal graft rejection, neovascularization following injury or infection, rubeosis, diabetic retinopathy, retrolental fibroplasia and neovascular glaucoma, corneal diseases and macular degeneration.

The methods of the present invention may be used to treat other angiogenesis-mediated disorders including hemangioma, solid tumors, leukemia, metastasis, telangiectasia, psoriasis, scleroderma, pyogenic granuloma, myocardial angiogenesis, Crohn's disease, plaque neovascularization, coronary collaterals, cerebral collaterals, arteriovenous malformations, ischemic limb angiogenesis, corneal diseases, retrolental fibroplasia, arthritis, diabetic neovascularization, peptic ulcer, Helicobacter related diseases, fractures, keloids, and vasculogenesis.

Specific disorders that can be treated, and compounds and compositions for use in the methods of the present invention, are described in more detail below.

### Ocular Disorders Mediated by Angiogenesis

Various ocular disorders are mediated by angiogenesis, and may be treated using the methods described herein. One example of a disease mediated by angiogenesis is ocular neovascular disease, which is characterized by invasion of new blood vessels into the structures of the eye and is the most common cause of blindness. In age-related macular degeneration, the associated visual problems are caused by an ingrowth of choroidal capillaries through defects in Bruch's membrane with proliferation of fibrovascular tissue beneath the retinal pigment epithelium. In the most severe form of age-related macular degeneration (known as "wet" ARMD) abnormal angiogenesis occurs under the retina resulting in irreversible loss of vision. The loss of vision is due to scarring of the retina secondary to the bleeding from the new blood vessels. Current treatments for "wet" ARMD utilize laser based therapy to destroy offending blood vessels.
However, this treatment is not ideal since the laser can permanently scar the overlying retina and the offending blood vessels often re-grow. An alternative treatment strategy for macular degeneration is the use of antiangiogenesis agents to inhibit the new blood vessel formation or angiogenesis which causes the most severe visual loss from macular degeneration.

Other conditions associated with or caused by angiogenic damage for which the present invention may be used include diabetic retinopathy, retinopathy of prematurity, corneal graft rejection, neovascular glaucoma and retrolental fibroplasia, diseases associated with corneal neovascularization including, but are not limited to, epidemic keratoconjunctivitis, Vitamin A deficiency, atopic keratitis, superior limbic keratitis, pterygium keratitis sicca, and periphigoid radial keratotomy, diseases associated with retinal/choroidal neovascularization including, but are not limited to, macular degeneration, presumed myopia, optic pits, chronic retinal detachment, hyperviscosity syndromes, trauma and post-laser complications. Other diseases which may be treated using the invention include, but are not limited to, diseases associated with rubeosis (neovascularization of the angle) and diseases caused by the abnormal proliferation of fibrovascular or fibrous tissue including all forms of proliferative vitreoretinopathy, neovascular glaucoma, retinoblastoma, retrolental fibroplasia, rubeosis, uveitis, and corneal graft neovascularization other eye inflammatory diseases, ocular tumors, and diseases associated with choroidal or iris neovascularization.

### Inflammation

The antibody molecules and methods of the invention may be used in the treatment of inflammation. By blocking the activity of CatS, the antibody molecules may prevent proper antigen presentation in 'inflammed' cells and thus dampen the inflammatory effects.

In such an embodiment, the antibody will ideally be taken into the cell to enter the lysosome. Thus targetting methods common in the art may be used. As shown in the Examples, from the pH binding experiments, the inventors have demonstrated that the antibody will bind even at pH 4.9, suggesting that it may be effective in the lysosome.

The methods of the invention may also be used to treat angiogenesis associated inflammation, including various forms of arthritis, such as rheumatoid arthritis and osteoarthritis.

Further, in these methods, treatment with combinations of the compounds described herein with other agents useful for treating the disorders is provided. Such agents include, for instance, cyclooxygenase-2 (COX-2) inhibitors, which are well known to those of skill in the art.

The blood vessels in the synovial lining of the joints can undergo angiogenesis. The endothelial cells form new vascular networks and release factors and reactive oxygen species that lead to pannus growth and cartilage destruction. These factors are believed to actively contribute to rheumatoid arthritis and also to osteoarthritis. Chondrocyte activation by angiogenic-related factors contributes to joint destruction, and also promotes new bone formation. The methods described herein can be used as a therapeutic intervention to prevent bone destruction and new bone formation.

Pathological angiogenesis is also believed to be involved with chronic inflammation. Examples of disorders that can be treated using the methods described herein include ulcerative colitis, Crohn's disease, bartonellosis, and atherosclerosis.

### Pharmaceutical Compositions

The antibody molecules and nucleic acids may be administered as a pharmaceutical composition. Pharmaceutical compositions according to the present invention, and for use in accordance with the present invention may comprise, in addition to active ingredients, a pharmaceutically acceptable excipient, a carrier, buffer stabiliser or other materials well known to those skilled in the art (see, for example, (Remington: the Science and Practice of Pharmacy, 21st edition, Gennaro AR, et al, eds., Lippincott Williams & Wilkins, 2005.).

Such materials may include buffers such as acetate, Tris, phosphate, citrate, and other organic acids ; antioxidants; preservatives; proteins, such as serum albumin, gelatin, or immunoglobulins ; hydrophilic polymers such aspolyvinylpyrrolidone ; amino acids such as glycine, glutamine, asparagine, histidine, arginine, or lysine ; carbohydrates; chelating agents; tonicifiers; and surfactants.

The pharmaceutical compositions may also contain one or more further active compound selected as necessary for the particular indication being treated, preferably with complementary activities that do not adversely affect the activity of the antibody molecule, nucleic acid or composition of the invention. For example, in the treatment of cancer, in addition to an anti CatS antibody molecule of the invention, the formulation may comprise an additional antibody which binds a different epitope on CatS, or an antibody to some other target such as a growth factor that e.g. affects the growth of the particular cancer, and/or a chemotherapeutic agent.

For example, in one embodiment, combination therapy employing a specific binding agent of the invention and an agent which inhibits, for example vascular endothelial growth factor (VEGF) may be used.

Suitably, combination therapy employing a specific binding agent of the invention and an agent which inhibits EGF receptor, PDGFβ, thrombin, bFGF, VEGFR kinase, fibroblast growth factor, tubulin, VEGF-A, or placental growth factor may be used.

In particular embodiments, combination therapy including at least an antibody molecule of the invention and two, three, or more agents in combination may be used.

In particular embodiments, a combination therapy employing a antibody molecule of the invention and an agent selected from Evizon™, PTK787, Retaane™, AG-13958, CAND5, Combretastatin™ or VEGF Trap™ may be used.

By employing combination therapy targeting at least two distinct pathways or mechanisms, improved efficacy, for example a synergistic effect, may be obtained.

The active ingredients (e.g. antibody molecules and/or chemotherapeutic agents) may be administered via microspheres, microcapsules liposomes, other microparticulate delivery systems. For example, active ingredients may be entrapped within microcapsules which may be prepared, for example, by coacervation techniques or by interfacial polymerization, for example, hydroxymethylcellulose or gelatinmicrocapsules and poly-(methylmethacylate) microcapsules, respectively, in colloidal drug delivery systems (for example, liposomes, albumin microspheres, microemulsions, nano-particles and nanocapsules) or in macroemulsions. For further details, see Remington: the Science and Practice of Pharmacy, 21st edition, Gennaro AR, et al, eds., Lippincott Williams & Wilkins, 2005.

Sustained-release preparations may be used for delivery of active agents. Suitable examples of sustained-release preparations include semipermeable matrices of solid hydrophobic polymers containing the antibody, which matrices are in the form of shaped articles, e. g. films, suppositories or microcapsules. Examples of sustained-release matrices include polyesters, hydrogels (for example, poly (2-hydroxyethyl-methacrylate), or poly (vinylalcohol)), polylactides (U. S. Pat. No. 3, 773, 919), copolymers of L-glutamic acid andy ethyl-Lglutamate,non-degradable ethylene-vinyl acetate, degradable lactic acid-glycolic acid copolymers, and poly-D- (-)-3-hydroxybutyric acid.

As described above nucleic acids of the invention may also be used in methods of treatment. Nucleic acid of the invention may be delivered to cells of interest using any suitable technique known in the art. Nucleic acid (optionally contained in a vector) may be delivered to a patient's cells using in vivo or ex vivo techniques. For in vivo techniques, transfection with viral vectors (such as adenovirus, Herpes simplex I virus, or adeno-associated virus) and lipid-based systems (useful lipids for lipid-mediated transfer of the gene are DOTMA, DOPE and DC-Chol, for example) may be used (see for example, Anderson et al., Science 256 : 808-813 (1992). See also WO 93/25673).

In ex vivo techniques, the nucleic acid is introduced into isolated cells of the patient with the modified cells being administered to the patient either directly or, for example, encapsulated within porous membranes which are implanted into the patient (see, e. g. U. S. Patent Nos. 4, 892, 538 and 5, 283, 187). Techniques available for introducing nucleic acids into viable cells may include the use of retroviral vectors, liposomes, electroporation, microinjection, cell fusion, DEAE-dextran, the calcium phosphate precipitation method, etc.

The antibody molecule, agent, product or composition may be administered in a localised manner to a tumour site or other desired site or may be delivered in a manner in which it targets tumour or other cells. Targeting therapies may be used to deliver the active agents more specifically to certain types of cell, by the use of targeting systems such as antibody or cell specific ligands. Targeting may be desirable for a variety of reasons, for example if the agent is unacceptably toxic, or if it would otherwise require too high a dosage, or if it would not otherwise be able to enter the target cells.

### Dose

The antibody molecules, nucleic acids or compositions of the invention are preferably administered to an individual in a "therapeutically effective amount", this being sufficient to show benefit to the individual. The actual dosage regimen will depend on a number of factors including the condition being treated, its severity, the patient being treated, the agent being used, and will be at the discretion of the physician.

The optimal dose can be determined by physicians based on a number of parameters including, for example, age, sex, weight, severity of the condition being treated, the active ingredient being administered and the route of administration.

As a rough guideline, doses of antibodies may be given in amounts of Ing/kg- 500mg/kg of patient weight.

### Assays

The antibody molecules of the present invention may further be used in diagnostic assays to determine the presence of cathepsin or cathepsin S expressing cells in a biological sample. Thus the antibody molecule of the invention may be used in a diagnostic method.

In one embodiment, the invention provides a method for determining the presence of cell expressing cathepsin S in a biological sample, said method comprising contacting the biological sample with an antibody molecule of the invention and determining binding of the antibody molecule to the biological sample, wherein binding of the antibody molecule to the biological sample relative to a control is indicative of the presence of cathepsin S.

The antibody molecules may also be used in the diagnosis of a variety of conditions and disorders associated with Cathepsin S expression or activity. Such methods form another aspect of the invention.

In one embodiment, the invention provides an assay method for detecting a tumour in a subject, comprising:
(a) contacting a biological sample from the subject with an antibody molecule of the invention
(b) determining a level of binding of the antibody molecule to the biological sample, wherein an elevated level of binding of the antibody molecule to the biological sample relative to a control sample is indicative of the presence of said disease.

The invention may be used to monitor disease progression, for example using biopsy samples at different times. In such embodiments, instead of comparing the expression of cathepsin S against a control sample from e.g. a different tissue source known not have enhanced cathepsin S expression, the expression of the cathepsin S is compared against a biological sample obtained from the same tissue at an earlier time point, for example from days, weeks or months earlier.

Any suitable biological sample may be used in the invention; the nature of the disease or condition may determine the nature of the sample which is to be used in the methods of the invention. The sample may be, for example, a sample from a tumour tissue biopsy, bone marrow biopsy or circulating cells in e.g. blood. Alternatively, e.g. where for example the methods are being used to diagnose or monitor a gastrointestinal tumour, tumour cells may be isolated from faeces samples. Other sources of biological sample may include plasma, serum, cerebrospinal fluid, urine, interstitial fluid, ascites fluid etc.

For example, solid tumour samples may be collected in complete tissue culture medium with antibiotics. Cells may be manually teased from the tumour specimen or, where necessary, are enzymatically disaggregated by incubation with collagenase/DNAse and suspended in appropriate media containing, for example, human or animal sera.

In other embodiments, biopsy samples may be isolated and frozen or fixed in fixatives such as formalin. The samples may then be tested for expression levels of genes at a later stage.

The invention further extends to diagnostic kits for use in the detection of cathepsin S. Thus, in one embodiment, the invention extends to a kit for the diagnosis of the presence of cathepsin S, the kit comprising one or more antibody molecules of the invention, or one or more polypeptides of the invention.

Any suitable assay methods and kits may be used. These include but are not be limited to ELISA, Immunohistochemistry, Electron Microscopy, Latex agglutination, lateral flow immunoassays, Immuno Blotting and Dip Stick Immuno testing.

The invention will now be described further in the following non-limiting examples. Reference is made to the accompanying drawings in which:
Figure 1 shows photographs of HMEC cells cultured in vehicle only control, an isotype control, an anti cathepsin S antibody (mAb1), or an anti cathepsin S antibody (mAb2);
Figure 1b illustrates graphs illustrating the inhibition of capillary cell branching observed in the presence of 1E11 (upper panel) or 1E4 (lower panel);
Figure 2a illustrates photographs of sections of aorta cultured in the presence of a control antibody and anti cathepsin S antibody 1E11 at 60, 300 and 600nM concentrations;
Figure 2b (top left) illustrates a graph summarising the effect of the antibody on vessel length as shown in Figure 2a;

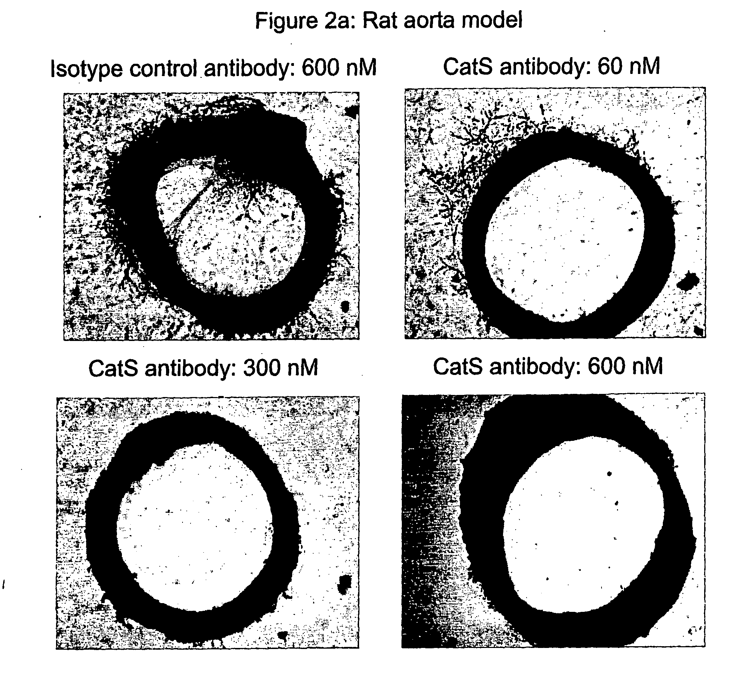

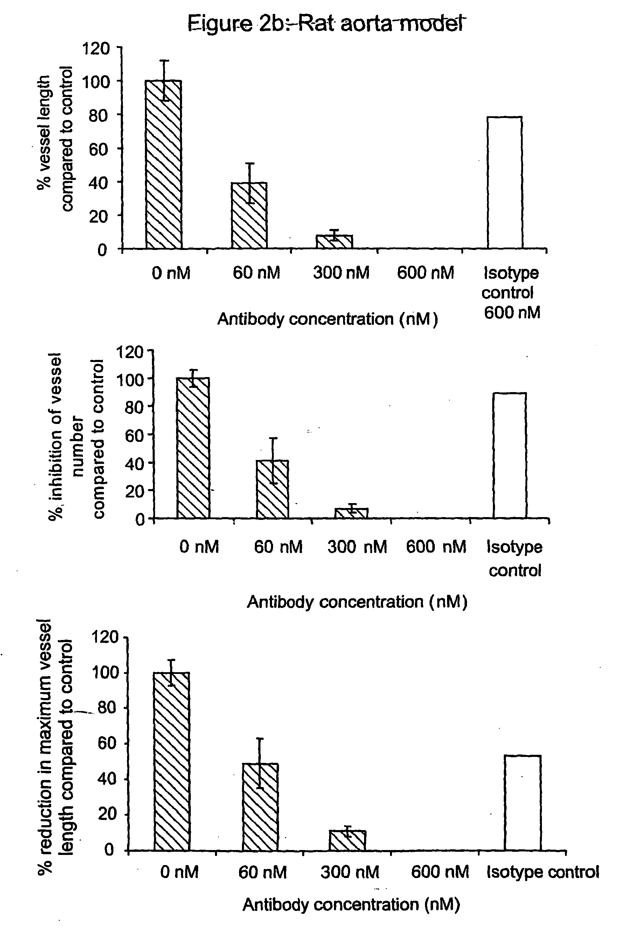

Figure 2b (top right) illustrates a graph summarising the effect of the antibody on vessel number as shown in Figure 2a;
Figure 2b (bottom) illustrates a graph summarising the effect of the antibody on maximum vessel length as shown in Figure 2a;

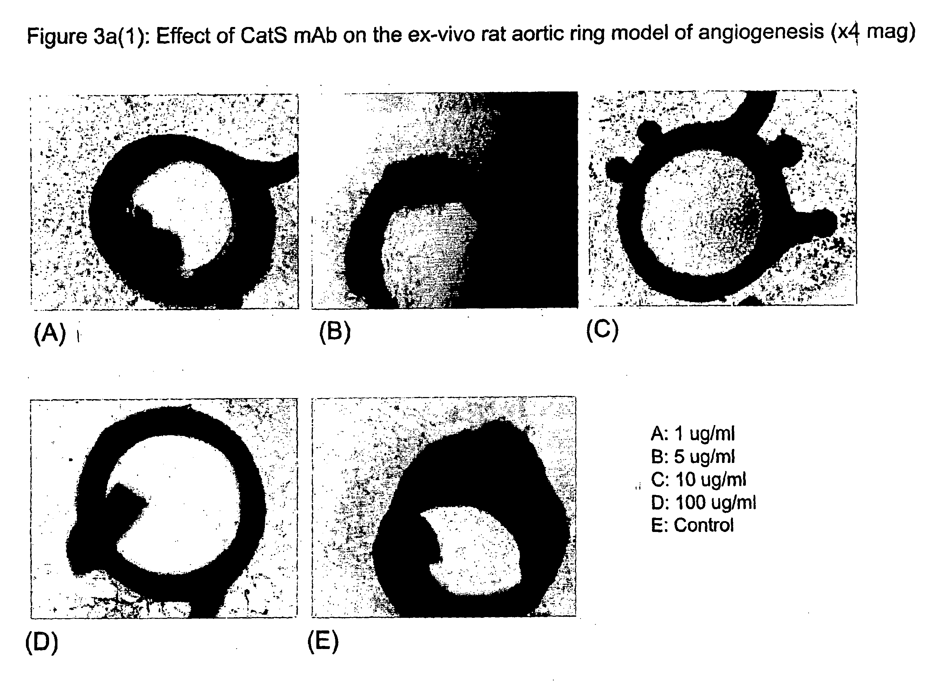

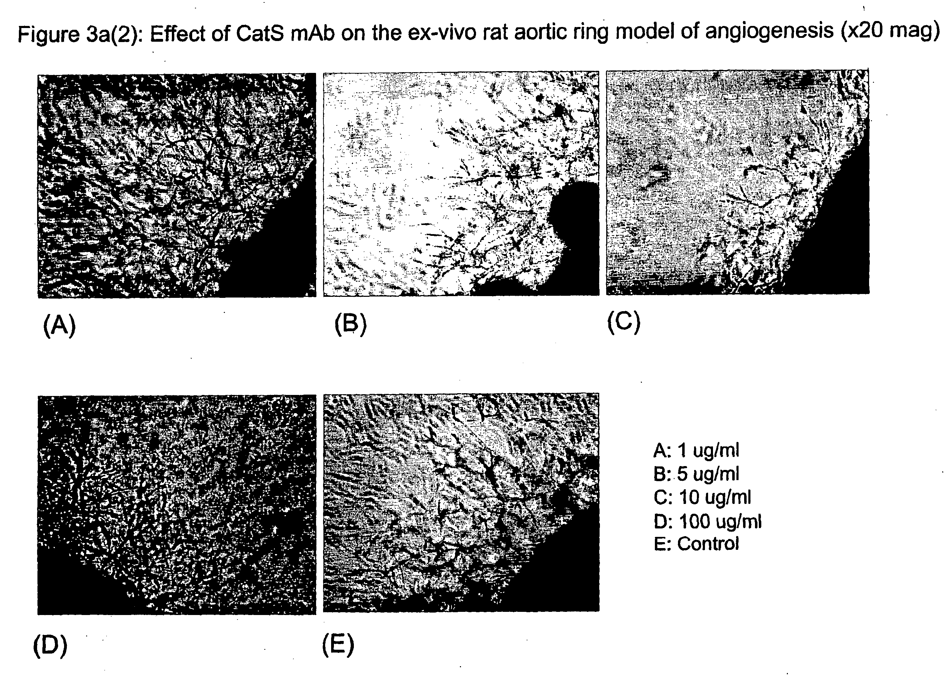

Figure 3a illustrates photographs of sections of aorta cultured in the presence of a control antibody and anti cathepsin S antibody IE11 at 1ug/ml, 5ug/ml, 10ug/ml, and 100ug/ml, 1ug/ml concentrations;

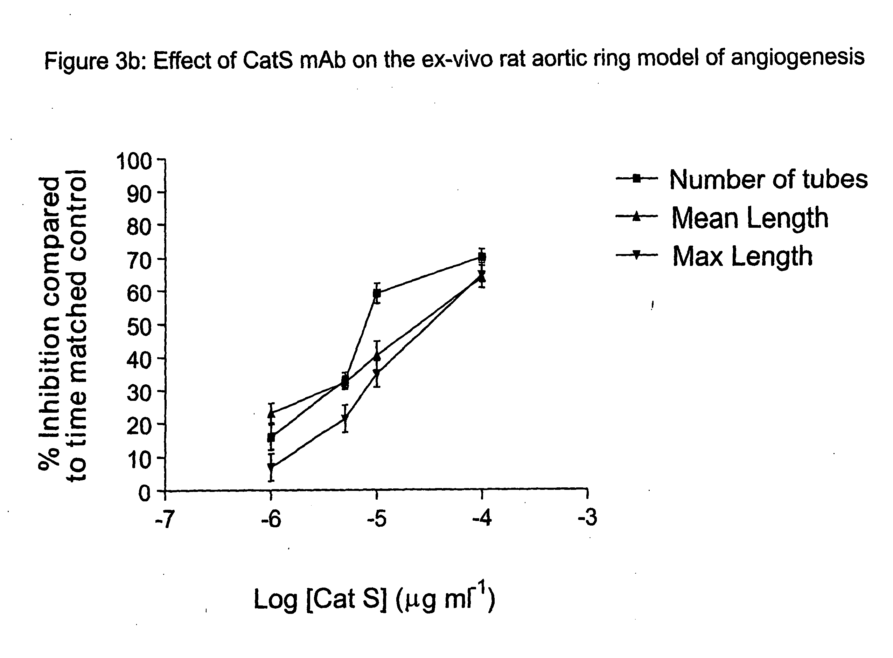

Figure 3b illustrates a graph summarising the effect of the antibody in the experiment illustrated in Figure 3a on number of tubules, mean tubule length and maximum tubule length;

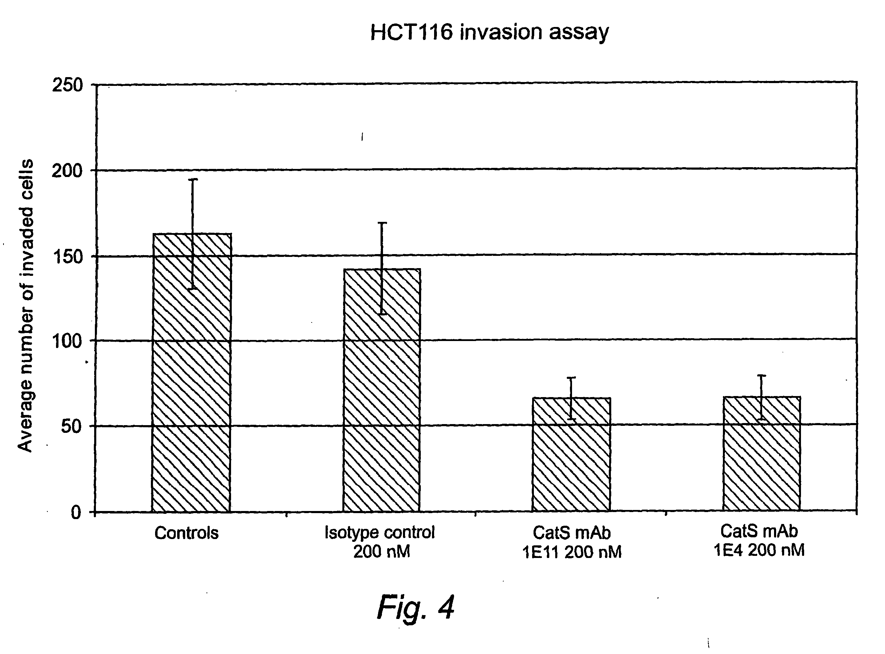

Figure 4 illustrates the results of an invasion assay demonstrating attenuation of HCT116 tumour invasion when treated with CatS 1E11 or 1E4;

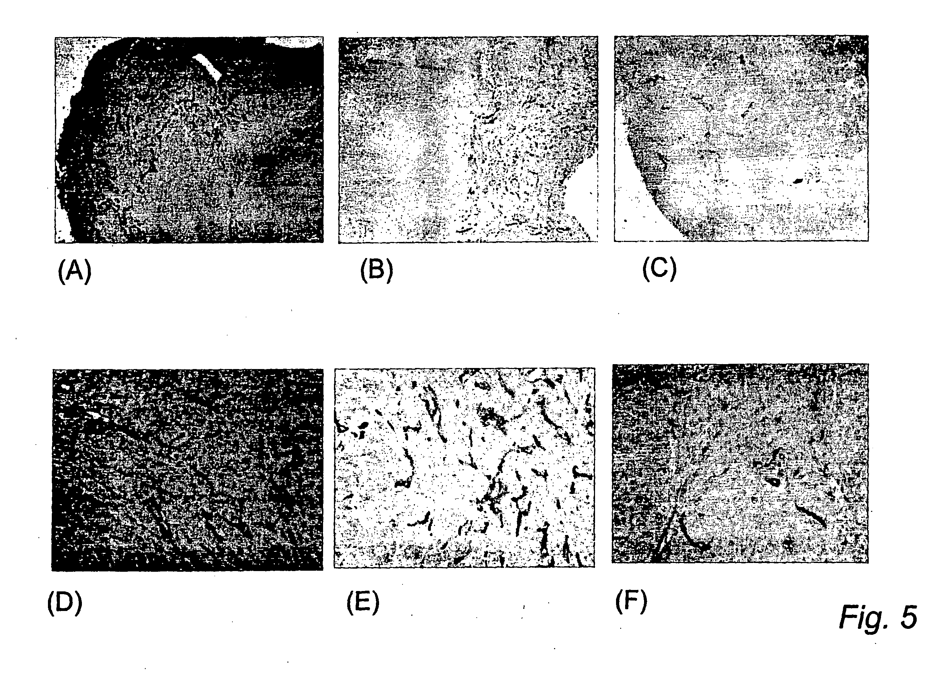

Figure 5 illustrates CD34 staining of control treated HCT116 tumours;

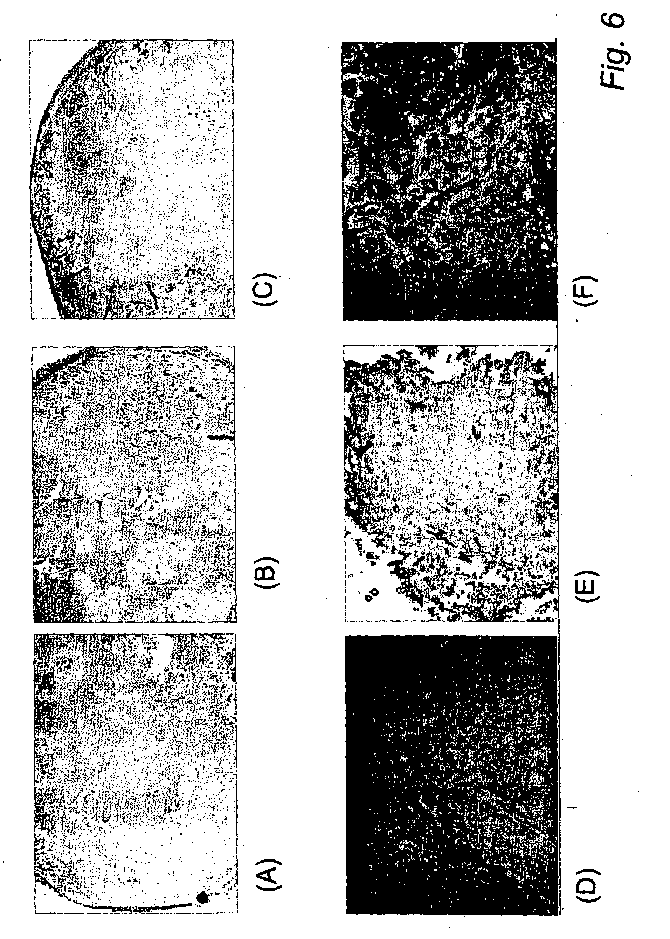

Figure 6 illustrates CD34 staining of CatS 1E11 treated HCT116 tumours;

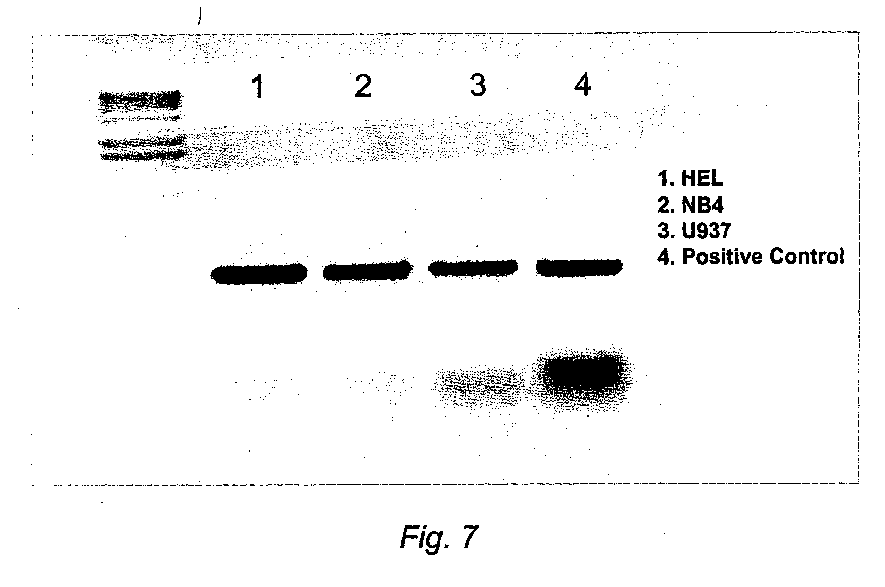

Figure 7 illustrates analysis of CatS RNA expression in leukaemia cell lines (HEL, NB4 & U937). RNA levels were analysed following 40 cycles of PCR to determine target expression; and

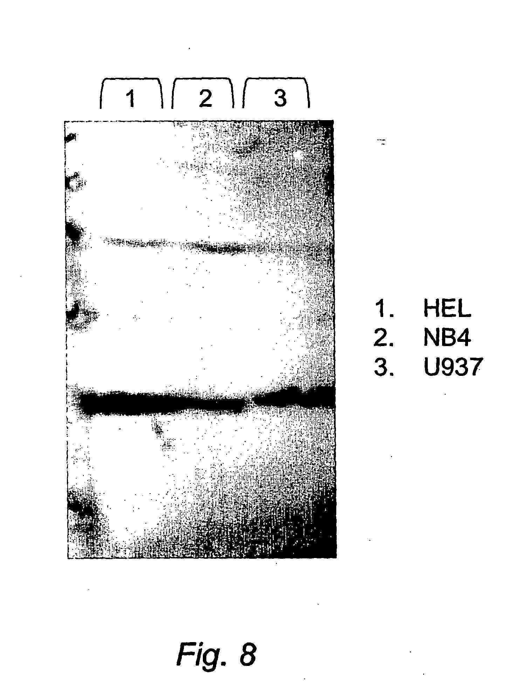

Figure 8 illustrates western blot analysis of CatS expression in leukaemia cell lines (HEL, NB4 & U937). Protein levels were analysed in whole cell lysates using 1E4 Anti CatS.

### Methods

### Capillary-Like Tube Formation Assay

The effect of the CatS mAb on endothelial cell tube formation was assessed as follows. Two hundred microliter of Matrigel (10 mg/ml) was applied to pre-cooled 48-well plates, incubated for 10 min at 4°C and then allowed to polymerize for 1 h at 37°C. Cells were suspended in endothelial growth cell medium MV (Promocell), containing 200 nM of the appropriate antibody. Five hundred microliter (1 × 10⁵ cells) were added to each well. As controls, cells were incubated with vehicle-only control medium containing the appropriate volumes of PBS. After 24 h incubation at 37°C and 5% CO2, cells were viewed using a Nikon Eclipse TE300 microscope.

### Cell viability and proliferation assays

Cytotoxic and proliferative effects of the CatS monoclonal antibody on U251mg astrocytoma cells can be tested as previously described. Briefly, cells are added to a final concentration of 1 × 10⁴ cells/200 µl per well of a 96-well microtiter plate (Corning Costar). Appropriate concentrations of monoclonal antibody (100 nM) or vehicle-only control media are added. Plates are incubated at 37°C and 5% CO₂ for 24, 48, 72 and 96 hrs respectively. After incubation, 10µl of 10 mg/ml MTT is added and incubated for a further 2 h at 37°C and 5% CO₂. The medium is carefully removed and formazan crystals dissolved in 100 µl/well of DMSO. Absorbance is measured as described above and the results expressed as the percentage of cell viability and proliferation relative to each vehicle-only control. All tests are performed in quadruplicate.

### Wound Assay

The *in vitro* migration assay used in these studies is a modified version of the method described by Ashton et al (1999). HMEC-1 is plated into individual chambers on a glass slide and grown to 90% confluence overnight. The medium is removed and the monolayer wounded. The monolayer is re-supplemented with fresh medium and the required volume of antibodies added to give the required final concentration.

Slides are removed at fixed time points until complete closure of the wound, then fixed in 4% PBS buffered paraformaldehyde. The extent of "wound" closure is blindly assessed microscopically by an independent investigator and quantified using a calibrated eyepiece graticule (1mm/100µm graduation) at 20x magnification (Olympus BX 50). The extent of closure in the antibody treated slides is compared to time matched sham treated controls and the % inhibition of wound closure compared to time matched controls calculated.

### Rat Aorta Model

Male Wistar rats are euthanised and the thoracic aorta is aseptically removed and sectioned into 1 cm thick rings. The rings are washed ten times in sterile medium to remove any bacteria and embedded into Matrigel on 24 well plates. The wells are supplemented with 2ml of medium and increasing concentrations of antibodies. The plate is incubated for 8 days and post incubation the Matrigel and rings are fixed in 4% PBS buffered paraformaldehyde and stored in PBS. The extent of vessel development is blindly assessed microscopically by an independent investigator and quantified using a calibrated eyepiece graticule (1mm/100)µm graduation) at 20x magnification (Olympus BX 50). The extent of vessel length, maximum vessel length and number of vessels in each field of view is measured and compared to time matched sham controls and the % inhibition calculated.

### PCR

RT-PCR was performed using a DNA Engine Tetrad 2 thermal cycler (Biorad). RNA was collected from leukaemia cell pellets using the RNA STAT-60 reagent (Tel-Test Friendswood, USA) according to the manufacturers instructions and cDNA synthesised using 1ug RNA and a reverse transcriptase kit (GIBCO Invitrogen, Paisley, UK). The primer sets used for RT-PCR were CatS forward primer 5'- ACT CAG AAT GTG AAT CAT GGT G-3' and CatS reverse primer 5'-TTC TTG CCA TCC GAA TAT ATC C-3'. Gene expression was analysed using a biomix PCR mixture (Bioline, UK) containing 25ul Biomix; 1.5µl forward primer; 1.5µl reverse primer; 2µl cDNA; 20µl dH₂O. PCR conditions consisted of an initial denaturation step of 95°C for 10 minutes, followed by either 40 cycles of 95°C for 30 sec; 55°C for 30 sec; 72°C for 90 sec, with a final extension of 72°C for 10 minutes. 5 µl of amplified product was loaded onto a 1.5% agarose gel (0.001% ethidium bromide) which was ran at 90V for 40 minutes prior to analysis on a UV box.

### Western Blotting

Cells were washed in PBS (5 minute centrifugation at 1500rpm) prior to lysis with RIPA buffer containing a protease inhibitor cocktail (Calbiochem, UK) as previously described. Whole cell lysates were loaded onto 12% SDS-PAGE gels at equal concentrations. Gels were run overnight at 50V prior to semi-dry transfer onto nitrocellulose membrane at 20V for 45 minutes (BioRad, UK). The nitrocellulose membrane was blocked using in PBS (3% Milk powder) for ∼1 hour, and washed x 3 with PBS (0.01% tween) for 5 minutes. The membrane was then probed with a 1 in 200 dilution of CatS Mab 1E4 Mab in PBS (3% Milk powder) for 1 hour. The membrane was washed x 3 with PBS (0.01% tween) for 10 minutes prior to the addition of a 1 in 5000 dilution of secondary goat anti mouse-HRP conjugated antibody (BioRad, UK) in PBS (3% Milk powder) for 1 hour. This was then washed three times in PBS (0.01% tween) for 10 minutes each. The membrane was 'developed' by ECL (enhanced chemi-luminescence) using the Super Signal kit (Pierce). The membrane was incubated in ECL solution for 5 minutes, prior to analysis using a kodak Gel logic 1500 imaging system (Kodak)

### Immunohistochemistry

Paraffin embedding of formalin-fixed xenograft tissue samples was performed as previously described. Immunostaining was performed using the avidin-horseradish peroxidase method (Vectorlabs ABC Elite Kit).

Briefly, sections were deparaffinised by passing from histoclear to alcohol to running water. Sections were then boiled in citrate buffer, pH6.0, for 22 min. Endogenous peroxidase activity was blocked by incubation in 3% H₂O₂, in methanol for 13 min. Incubation in 10% normal rabbit serum blocking solution was carried out for 1 hour at room temp. Sections were incubated with primary rat anti human CD34 (Abcam) at a 2ug/ml concentration at 4°C overnight. Appropriate isotype controls were used at the same dilutions as primary antibodies.

For peroxidase staining, sections were incubated with biotinylated rabbit anti rat secondary antibody (Vector Laboratories) for 30 min at room temp followed by incubation with the Vectastain Elite ABC reagent (Vector Laboratories) for a further 30 min at room temp. For visualisation sections were stained with 3,3'-diaminobenzidine and counterstained with Gill's II hematoxylin solution. Sections were analyzed using a Nikon Eclipse 80i camera

### Invasion Assay

In-vitro invasion assays were performed using a modified Boyden chamber with 12 µm pore membranes. The upper membrane surface was coated with Matrigel (100 µg/cm²) and allowed to dry overnight in a laminar flow hood. Cells were added (5 x 10⁵ cells in 500 µl of serum free media) in the presence of pre-determined concentrations of the appropriate antibody and recombinant protein. Fresh complete media was added to the lower chambers (1.5 mls), supplemented with the same concentration of the antibody or recombinant protein as was applied to the corresponding well above. All assays were carried out in triplicate and invasion plates were incubated at 37 °C and 5% CO₂ for 24 hours.

Cells remaining on the upper surface of the membrane were removed by wiping with cotton tips and cells which had invaded through were fixed in Carnoy's fixative for 15 minutes. After drying, the nuclei of the invaded cells were stained with Hoechst 33258 (50 ng/ml) in PBS for 30 minutes at room temperature. The chamber insert was washed twice in PBS, mounted in PermaFluor mounting medium and invaded cells were viewed with a Nikon Eclipse TE300 fluorescent microscope. Ten digital images of representative fields from each of the triplicate membranes were taken using a Nikon DXM1200 digital camera at magnification of x20. The results were analysed using Lucia GF 4.60 by Laboratory Imaging and were expressed as a percentage of invaded cells compared to controls.

### Results and Discussion

### Example 1. CatS Antibodies can inhibit tube-like formation in human endothelial cells

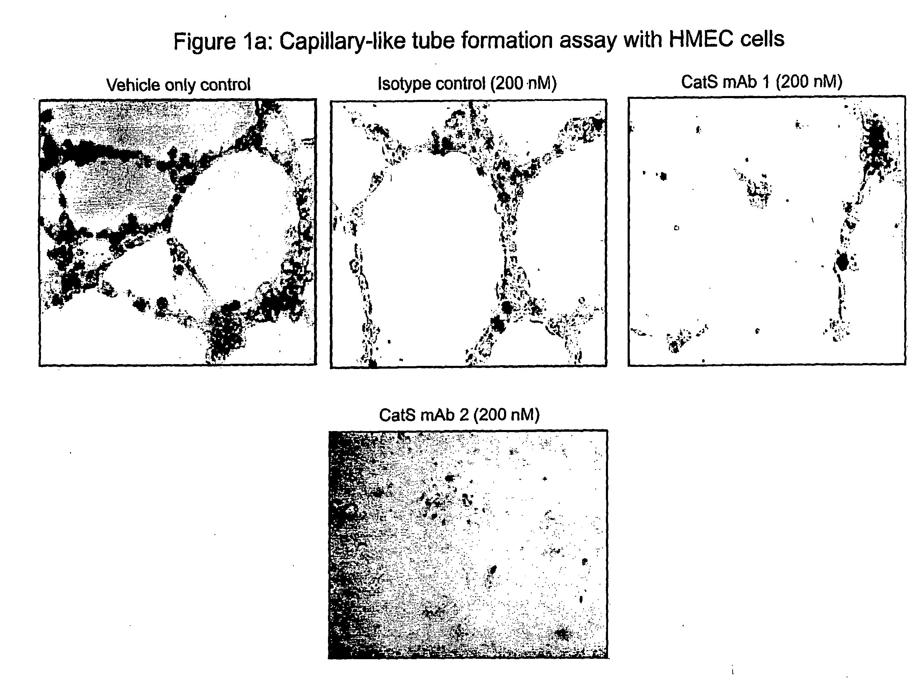

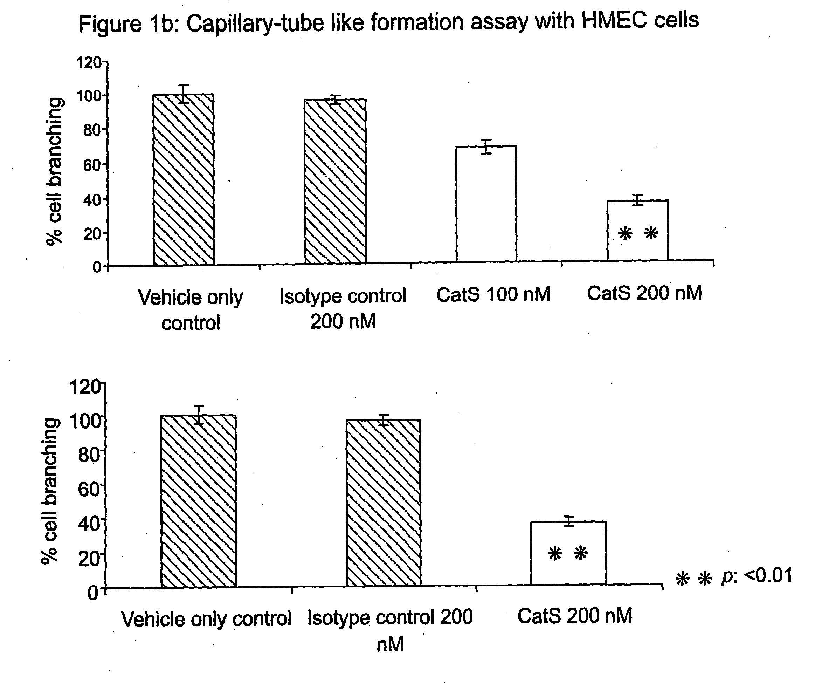

Using the Matrigel morphogenesis assay described by (Grant DS, Tashiro K, Segui-Real B, Yamada Y, Martin GR, Kleinman HK.Two different laminin domains mediate the differentiation of human endothelial cells into capillary-like structures in vitro. Cell. 1989 Sep 8;58(5):933-43.), capillary-tubule formation assays were performed with human microvascular endothelial cells (HMECs) cultured on Matrigel enabling the endothelial cells form tube-like structures, with invasive sprouts extending from individual cells to form contacts with nearby endothelial cells. Figure 1a illustrates the results when the HMEC cells were cultured in the presence of two CatS antibodies(1E11 and 1E4) or isotype control. Extensive tube-like structures are evident in the vehicle-only control and isotype control antibody (200 nM) panels; however this tube formation is almost completely abolished in the presence of the 1E11 or 1E4 antibodies (200 nM). These results were then quantified as shown in Figure 1b.

Both antibodies have been previously shown to bind specifically to CatS with no cross-reactivity with other cathepsins, in particular those with the greatest homology to CatS. Antibodies 1E11 and 1E4 have both been characterised for their ability to inhibit the catalytic activity of CatS; 1E11 can specifically inhibit the activity of CatS whereas 1E4 has no discernable effect. Therefore, the results from the capillary-tube assay would suggest that the sequestering of active CatS secreted from the endothelial cells by either an inhibitory or non-inhibitory CatS mAb is sufficient to prevent the migration and arrangement of the endothelial cells into tube-like structures.

### Example 2. CatS antibodies can inhibit tube like formation in rat aortic arch ex vivo model

To evaluate further the role of CatS in angiogenesis, an *ex vivo* rat aortic arch assay was performed. Sections of the aorta (1 mm) were cultured within a thin layer of Matrigel in the presence of the inhibitory antibody and appropriate controls. The formation of tube-like vessels from the aorta were monitored and quantified after 7 days by measuring the reduction in the number of vessels, mean vessel length and maximum vessel length compared to controls.

Figure 2 illustrates the significant inhibition of tube formation in the presence of the CatS 1E11 antibody. Photographs of the ring segments are shown in Figure 2a with the results summarised in Figure2b. Incubation of the rat aortic ring segments with up to 600nM of 1E11 resulted in greater than 80% reduction in total vessel number, mean vessel length and maximum vessel length (figure 2b).

Figure 3 illustrates the significant inhibition of tube formation in a repeat experiment the presence of the CatS 1E11 antibody. Photographs of the ring segments are shown in Figures 3a and 3b with the results summarised in Figure 3c. Incubation of the rat aortic ring segments with up to 10 µg/ml of 1E11 resulted in a 70% reduction in total vessel number and a 60% reduction in both mean vessel length and maximum vessel length.

### Example 3

To investigate the effect of the CatS antibodies on tumour invasion, an invasion assay was performed as described above using HCT116 cells. The results are shown in Figure 4. As can be seen, tumour invasion was significantly attenuated in HCT116 cells treated with the CatS 1E11 antibody and in HCT116 cells treated with the 1E4 CatS antibody.

### Example 4 Effect of treatment on vasculature of HCT116 tumours treated with CatS antibody

The effect of treatment on the vasculature of HCT116 tumours treated with CatS antibody was investigated by staining CatS 1E11 treated HCT 116 tumours using CD34 and comparing with HCT 116 tumours treated with a control antibody. The results are shown in Figure 5 and Figure 6. As can be seen from Figure 5, in the control treated tumours, vasculature was noted evidently at the periphery of the tumour (A-C (X4)). Large number of small, functionally limited blood vessels present, with some larger vessels also determined (D-E (X20)). However, as shown in Figure 6, in the CatS 1E11 treated HCT116 tumours, vasculature at the periphery of the tumours was greatly reduced (A-C (X4)) and the number of vessels presented was lower compared to the control treated tumours.

### Example 5 CatS 1E4 bind CatS from leukaemia cell lines

CatS RNA was amplified from three different leukaemia cell lines and a positive control. RNA levels were analysed following 40 cycles of PCR to determine target expression. As can be seen from Figure 7, CatS RNA was expressed by each of the three leukaemia cell lines.

Further a Western blot was performed on whole cell lysates of the three leukaemia cell lines using the 1E4 antibody. As shown in Figure 8, the CatS 1E4 binds to the Cat S protein, showing that it can be used to target cathepsin S in leukaemia cells.

### Example 6 Identification of the cathepsin S binding region to which the 1E4 antibody specifically binds

CatS specific primers were designed to contain a 5' T7 promoter region and either a 5' or 3' hexahistidine tag. Using standard conditions, these primers were used to amplify multiple different regions of the CatS CDS before analysis by agarose electrophoresis. Neat, unpurified PCR products were the added to 50 µl aliquots of wheat germ cell-free lysate(Rapid Translation System, Roche) and incubated overnight at room temperature. These protein lysates were then electrophoresed and western blotted using standard techniques.

After blocking in 3% milk powder in PBS, pH 7.4 (w/v), CatS antibodies (1 in 200 dilution in PBS) were incubated overnight at 4 °C. Specific binding of the CatS antibodies to the blot was visualised by washing and detection with a secondary anti-mouse - HRP conjugate by chemoillumenscence following standard protocols. Verification of protein expressed was afforded by re-probing of these blots with anti-histidine tag monoclonal antibody -HRP conjugate (Sigma-Aldrich).

By determining to which of the multiple samples the CatS antibodies bound, the binding region was identified as having the amino acid sequence as shown as Sequence ID No: 1:
ELPYGREDVLKEAVANKGPVSVGVDARHP

Various modifications and variations to the described embodiments of the inventions will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes of carrying out the invention which are obvious to those skilled in the art are intended to be covered by the present invention.

## Claims

1. An isolated polypeptide, wherein said polypeptide consists of a polypeptide sequence having at least 90% homology to Sequence ID No: 1, wherein Sequence ID NO:1 is a region of cathepsin S.

2. The isolated polypeptide according to claim 1, wherein the said polypeptide consists of a polypeptide sequence having the amino acid sequence shown as Sequence ID No: 1.

3. An antibody molecule which specifically binds cathepsin S and which inhibits angiogenesis but does not inhibit the proteolytic activity of cathepsin S, wherein said antibody molecule has binding specificity for the polypeptide of claim 1 or claim 2.

4. The antibody molecule according to claim 3, or a nucleic acid encoding said antibody molecule for use in therapy.

5. The use of an antibody molecule or nucleic acid encoding the antibody molecule in the preparation of a medicament for the treatment of cancer, an inflammatory condition, an ocular disease, atherosclerosis, a neurodegenerative disorder, an autoimmune disorder, hemangioma, a solid tumor, leukemia, telangiectasia, psoriasis, scleroderma, pyogenic granuloma, myocardial angiogenesis, Crohn's disease, plaque neovascularization, coronary collaterals, cerebral collaterals, arteriovenous malformations, ischemic limb angiogenesis, arthritis, diabetic neovascularization, peptic ulcer, Helicobacter related disease, a fracture, keloids, and vasculogenesis, ulcerative colitis, or bartonellosis, wherein said antibody molecule is the antibody molecule according to claim 3.

6. The use according to claim 5, wherein said condition is cancer, an inflammatory condition, an ocular disease, or atherosclerosis.

7. A pharmaceutical composition comprising the antibody molecule according to claim 3 or a nucleic acid molecule encoding said antibody molecule.

8. The pharmaceutical composition according to claim 7, wherein said composition further comprises an inhibitor of VEGF, wherein said inhibitor of VEGF is an anti VEGF antibody, PTK787, anecortave , AG-13958, CAND5, or aflibercept.

9. The pharmaceutical composition according to claim 7 or claim 8, wherein said composition further comprises an anti EGF receptor antibody.

10. A method for determining the presence of cathepsin S in a biological sample, said method comprising contacting the biological sample with an antibody molecule according to claim 3 and determining binding of the antibody molecule to the biological sample, wherein binding of the antibody molecule to the biological sample relative to a control is indicative of the presence of cathepsin S.

11. An assay method for detecting a tumour in a subject, comprising:
(a) contacting a biological sample from the subject with an antibody molecule according to claim 3, and
(b) determining a level of binding of the antibody molecule to the biological sample, wherein an elevated level of binding of the antibody molecule to the biological sample relative to a control sample is indicative of the presence of a tumour.

## Patentansprüche

1. Ein isoliertes Polypeptid, wobei das Polypeptid aus einer Polypeptidsequenz besteht, die mindestens 90 % Homologie zu SEQ in NO: 1 aufweist, wobei SEQ ID NO: 1 ein Bereich von Cathepsin S ist.

2. Isoliertes Polypeptid gemäß Anspruch 1, wobei das Polypeptid aus einer Polypeptidsequenz besteht, die die als SEQ ID NO: 1 gezeigte Aminosäuresequenz aufweist.

3. Ein Antikörpermolekül, das Cathepsin S spezifisch bindet und das Angiogenese inhibiert, die proteolytische Aktivität von Cathepsin S jedoch nicht inhibiert, wobei das Antikörpermolekül eine Bindungsspezifität für das Polypeptid gemäß Anspruch 1 oder Anspruch 2 aufweist.

4. Antikörpermolekül gemäß Anspruch 3 oder eine Nukleinsäure, die das Antikörpermolekül zur Verwendung bei einer Therapie codiert.

5. Verwendung eines Antikörpermoleküls oder einer das Antikörpermolekül codierenden Nukleinsäure bei der Zubereitung eines Medikaments zur Behandlung von Krebs, einem entzündlichen Zustand, einer okularen Krankheit, Atherosklerose, einer neurodegenerativen Erkrankung, einer Autoimmunerkrankung, Hämangiom, einem soliden Tumor, Leukämie, Telangiektasie, Psoriasis, Sclerodermia, Granuloma pediculatum, myokardialer Angiogenese, Morbus Crohn, Plaque-Revaskularisation, koronaren Kollateralen, zerebralen Kollateralen, arteriovenösen Fehlbildungen, Angiogenese in ischämischen Extremitäten, Arthritis, diabetischer Revaskularisation, Ulcus pepticum, helicobacterbedingter Erkrankung, einem Bruch, Keloiden und Vaskulogenese, Colitis ulcerosa oder Bartonellose, wobei das Antikörpermolekül das Antikörpermolekül gemäß Anspruch 3 ist.

6. Verwendung gemäß Anspruch 5, wobei der Zustand Krebs, ein entzündlicher Zustand, eine okulare Krankheit oder Atherosklerose ist.

7. Eine pharmazeutische Zusammensetzung, die das Antikörpermolekül gemäß Anspruch 3 oder ein das Antikörpermolekül codierendes Nukleinsäuremolekül beeinhaltet.

8. Pharmazeutische Zusammensetzung gemäß Anspruch 7, wobei die Zusammensetzung ferner einen inhibitor von VEGF beinhaltet, wobei der Inhibitor von VEGF ein Anti-VEGF-Antikörper, PTK787, Anecortave, AG-13958, CAND5 oder Aflibercept ist.

9. Pharmazeutische Zusammensetzung gemäß Anspruch 7 oder Anspruch 8, wobei die Zusammensetzung ferner einen Anti-EGF-Rezeptor-Antikörper beinhaltet.

10. Ein Verfahren zum Bestimmen der Anwesenheit von Cathepsin S in einer biologischen Probe, wobei das Verfahren das In-Kontakt-Bringen der biologischen Probe mit einem Antikörpermolekül gemäß Anspruch 3 und das Bestimmen des Bindens des Antikörpermoleküls an die biologische Probe beinhaltet, wobei das Binden des Antikörpermoleküls an die biologische Probe relativ zu einer Kontrolle auf die Anwesenheit von Cathepsins S hinweist.

11. Ein Assay-Verfahren zum Erkennen eines Tumors bei einer Testperson, das Folgendes beinhaltet:
(a) In-Kontakt-Bringen einer biologischen Probe von der Testperson mit einem Antikörpermolekül gemäß Anspruch 3 und
(b) Bestimmen eines Grads des Bindens des Antikörpermoleküls an die biologische Probe, wobei ein erhöhter Grad des Bindens des Antikörpermoleküls an die biologische Probe relativ zu einer Kontrollprobe auf die Anwesenheit eines Tumors hinweist.

## Revendications

1. Un polypeptide isolé, dans lequel ledit polypeptide consiste en une séquence polypeptidique ayant au moins 90 % d'homologie avec la Séquence ID No: 1, où la Séquence ID No: 1 est une région de la cathepsine S.

2. Le polypeptide isolé selon la revendication 1, dans lequel ledit polypeptide consiste en une séquence polypeptidique ayant la séquence d'acides aminés montrée en tant que Séquence ID No : 1.

3. Une molécule d'anticorps qui lie spécifiquement la cathepsine S et qui inhibe l'angiogenèse mais qui n'inhibe pas l'activité protéolytique de la cathepsine S, dans laquelle ladite molécule d'anticorps possède une spécificité de liaison pour le polypeptide de la revendication 1 ou la revendication 2.

4. La molécule d'anticorps selon la revendication 3, ou un acide nucléique codant pour ladite molécule d'anticorps pour une utilisation en thérapie.

5. L'utilisation d'une molécule d'anticorps ou d'un acide nucléique codant pour la molécule d'anticorps dans la préparation d'un médicament pour le traitement de cancer, d'un état inflammatoire, d'une maladie oculaire, de l'athérosclérose, d'un trouble neurodégénératif, d'un trouble auto-immun, d'hémangiome, d'une tumeur solide, de la leucémie, de la télangiectasie, du psoriasis, de la sclérodermie, de granulome pyogénique, de l'angiogenèse myocardiale, de la maladie de Crohn, de la néovascularisation des plaques, des collatéraux coronaires, des collatéraux cérébraux, de malformations artérioveineuses, de l'angiogenèse du membre ischémique, de l'arthrite, de la néovascularisation diabétique, de l'ulcère peptique, de maladie liée à Helicobacter, d'une fracture, des chéloïdes, et de la vasculogenèse, de la colite ulcéreuse, ou de la bartoriellose, dans laquelle ladite molécule d'anticorps est la molécule d'anticorps selon la revendication 3.

6. L'utilisation selon la revendication 5, dans laquelle ledit état est un cancer, un état inflammatoire, une maladie oculaire, ou l'athérosclérose.

7. Une composition pharmaceutique comprenant la molécule d'anticorps selon la revendication 3 ou une molécule d'acide nucléique codant pour ladite molécule d'anticorps.

8. La composition pharmaceutique selon la revendication 7, dans laquelle ladite composition comprend de plus un inhibiteur de VEGF, dans laquelle ledit inhibiteur de VEGF est un anticorps anti-VEGF, PTK787, l'anecortave, A-13958, CANE5, ou l'aflibercept.

9. La composition pharmaceutique selon la revendication 7 ou la revendication 8, dans laquelle ladite composition comprend de plus un anticorps de récepteur anti-EGF.

10. Une méthode pour déterminer la présence de cathepsine S dans un échantillon biologique, ladite méthode comprenant le fait de mettre en contact l'échantillon biologique avec une molécule d'anticorps selon la revendication 3 et de déterminer la liaison de la molécule d'anticorps à l'échantillon biologique, dans laquelle la liaison de la molécule d'anticorps à l'échantillon biologique relativement à un témoin est indicatrice de la présence de cathepsine S.

11. Une méthode d'épreuve pour détecter une tumeur chez un sujet, comprenant le fait de:
(a) mettre en contact un échantillon biologique prélevé chez le sujet avec une molécule d'anticorps selon la revendication 3, et
(b) déterminer un niveau de liaison de la molécule d'anticorps à l'échantillon biologique, dans laquelle un niveau élevé de liaison de la molécule d'anticorps à l'échantillon biologique relativement à un échantillon témoin est indicatrice de la présence d'une tumeur.
